# NEUE EUROPÄISCHE PATENTSCHRIFT

(11) **EP 1 214 945 B2**
(45) Veröffentlichungstag und Bekanntmachung des Hinweises auf die Entscheidung über den Einspruch: **10.10.2018**
(45) Hinweis auf die Patenterteilung: 08.06.2005
(21) Anmeldenummer: 02003683.6
(22) Anmeldetag: 29.01.2000
(51) Int. Cl.: C12N 15/11, A61K 31/713

(54) **VERFAHREN UND MEDIKAMENT ZUR HEMMUNG DER EXPRESSION EINES VORGEGEBENEN GENS**
METHOD AND MEDICAMENT FOR INHIBITING THE EXPRESSION OF A DEFINED GENE
METHODE ET MEDICAMENT DESTINES A INHIBER L'EXPRESSION D'UN GENE DONNE

(30) Priorität: 30.01.1999 DE 19903713; 24.11.1999 DE 19956568
(43) Veröffentlichungstag der Anmeldung: 19.06.2002
(62) Teilanmeldung aus: 00910510.7
(73) Patentinhaber: Alnylam Europe AG, 95326 Kulmbach (DE)
(72) Erfinder: Kreutzer, Roland Dr., 95466 Weidenberg (DE); Limmer, Stefan Dr., 95326 Kulmbach (DE)
(74) Vertreter: Vossius & Partner Patentanwälte Rechtsanwälte mbB

(56) Entgegenhaltungen:
- WO-A-92/19732
- WO-A-98/05770
- WO-A-99/32619
- UHLMANN E ET AL: "ANTISENSE OLIGONUCLEOTIDES: A NEW THERAPEUTIC PRINCIPLE" CHEMICAL REVIEWS,US,AMERICAN CHEMICAL SOCIETY. EASTON, Bd. 90, Nr. 4, 1. Juni 1990 (1990-06-01), Seiten 543-584, XP000141412 ISSN: 0009-2665
- MADHUR K. ET AL.: "Antisense RNA : function and fate of duplex RNA in cells of higher eukaryotes." MICROBIOLOGY AND MOLECULAR BIOLOGY REVIEWS, Bd. 62, Dezember 1998 (1998-12), Seiten 1415-1434, XP000909741

## Beschreibung

Die Erfindung betrifft Verfahren zur Hemmung der Expression eines vorgegebenen Zielgens in einer Zelle in vitro. Sie betrifft ferner ein Medikament und eine Verwendung doppelsträngiger Oligoribonukleotide.

Ein solches Verfahren ist aus der nachveröffentlichten WO 99/32619 bekannt. Das bekannte Verfahren zielt auf die Hemmung der Expression von Genen in Zellen von Invertebraten ab. Dazu ist es erforderlich, daß das doppelsträngige Oligoribonukleotid eine zum Zielgen identische Sequenz mit einer Länge von mindestens 50 Basen aufweist. Zur Erzielung einer effizienten Hemmung ist eine Länge der identischen Sequenz von 300 bis 1000 Basenpaare erforderlich. Der Herstellungsaufwand eines solchen Oligoribonukleotids ist hoch.

Aus der WO 99/53050 ist ein Verfahren zur Hemmung der phänotypischen Expression einer Nukleinsäure in einer eukaryotischen Zelle bekannt. In die eukaryotische Zelle kann ein chimeres RNA-Molekül eingeführt werden. Eine doppelsträngige RNA (dsRNA) wird entweder in Zellen durch Transkription von in die Zellen eingeführten chimeren DNA-Molekülen gebildet oder als einzelsträngige RNA mit Haarnadelschleifenstruktur in die Zellen eingeführt.

Die WO 99/15682 betrifft ein Verfahren zur Hemmung der Expression einer Zielnukleotidsequenz in einer Pflanze. Dazu wird in das Zytoplasma einer Zelle ein aus DNA oder RNA bestehendes einzelsträngiges oder doppelsträngiges Nukleinsäurekonstrukt eingeführt. Das Nukleinsäurekonstrukt kodiert für eine Sequenz, die identisch ist mit der Zielnukleotidsequenz oder dem dazu komplementären Strang. Die Zielnukleotidsequenz ist dabei in einem ersten Teil der Pflanze vorhanden, während das Nukleinsäurekonstrukt in das Zytoplasma einer Zelle in einem zweiten Teil der Pflanze eingeführt wird.

Gemäß Fire, A., "RNA-triggered gene silencing", TIG September 1999, Vol. 15, Nr. 9, Seiten 358 bis 363 ist es unwahrscheinlich, dass die einfachen für Nichtwirbeltier- und Pflanzensysteme verwendeten Protokolle in Säugerzellen wirksam sind. Säugetiere zeigen eine heftige Reaktion auf dsRNA. Eine Komponente dieser Reaktion ist Proteinkinase (PKR). Jede genspezifische dsRNA-Antwort müsste in Säugern im Schatten der PKRinduzierten Gesamtantwort oder in Zellen oder unter Bedingungen stattfinden, bei denen PKR weniger wirksam ist. Zur Umgehung einer Beeinträchtigung durch das PKR-System werden chemische Modifikationen an der dsRNA vorgeschlagen, die noch eine Wirkung der dsRNA bei der genspezifischen Interferenz ermöglichen, ohne die PKR-Antwort auszulösen.

Die durch die Aktivierung von PKR durch dsRNA ausgelösten zellulären Mechanismen sind z.B. aus Clemens, J. A., PKR-A Protein Kinase Regulated by Double-stranded RNA, Int. J. Biochem. Cell Biol. (1997) Band 29, Nr. 7, Seiten 945-949 bekannt.

Die WO 92/19732 offenbart ein Antisinn-Agens mit topologisch geschlossener einzelsträngiger Struktur. Innerhalb dieser Struktur kann es einen selbstkomplementären doppelsträngigen Bereich geben. Dieser Bereich kann durch eine Wechselwirkung mit Proteinen wirken, welche eine Affinität für diesen Bereich aufweisen.

Die WO 98/05770 offenbart eine einzelsträngige Antisinn-RNA mit einer Haarnadelschleifenstruktur.

Die DE 196 31 919 C2 beschreibt eine Anti-Sinn-RNA mit besonderen Sekundärstrukturen, wobei die Anti-Sinn-RNA in Form eines sie kodierenden Vektors vorliegt. Bei der Anti-Sinn-RNA handelt es sich um ein RNA-Molekül, das komplementär zu Bereichen der mRNA ist. Durch Bindung an diese Bereiche wird eine Hemmung der Genexpression bewirkt. Diese Hemmung kann insbesondere zur Diagnose und/oder Therapie von Erkrankungen, z.B. Tumorerkrankungen oder viralen Infektionen, eingesetzt werden. - Die Anti-Sinn-RNA muß nachteiligerweise in einer Menge in die Zelle eingebracht werden, die mindestens genauso groß wie die Menge der mRNA ist. Die Wirksamkeit der bekannten Anti-Sinn-Verfahren ist nicht besonders hoch.

Aus der US 5,712,257 ist ein Medikament bekannt, das fehlgepaarte doppelsträngige RNA (dsRNA) und biologisch aktive fehlgepaarte Bruchstücke von dsRNA in Form eines ternären Komplexes mit einem oberflächenaktiven Mittel enthält. Die dabei verwendete dsRNA besteht aus synthetisch hergestellten Nukleinsäureeinzelsträngen ohne definierte Basensequenz. Die Einzelstränge gehen nicht-reguläre, sogenannte "Nicht-Watson-Crick"-Basenpaarungen miteinander ein, so daß fehlgepaarte Doppelstränge gebildet werden. Die bekannte dsRNA dient zur Hemmung der Vermehrung von Retroviren, wie HIV. Die Vermehrung des Virus kann gehemmt werden, wenn nicht-sequenzspezifische dsRNA in die Zellen eingebracht wird. Es kommt dabei zu einer Induktion von Interferon, wodurch die Virusvermehrung gehemmt werden soll. Der hemmende Effekt bzw. die Wirksamkeit dieses Verfahrens ist gering.

Aus Fire, A. et.al, NATURE, Vol. 391, pp. 806 ist es bekannt, daß dsRNA, deren einer Strang abschnittsweise komplementär zu einem zu hemmenden Gen eines Fadenwurms ist, die Expression dieses Gens mit einer hohen Wirksamkeit hemmt. Es wird die Auffassung vertreten, daß die besondere Wirksamkeit der verwendeten dsRNA in Zellen des Fadenwurms nicht auf dem Anti-Sinn-Prinzip beruht, sondern möglicherweise auf katalytische Eigenschaften der dsRNA bzw. durch sie induzierte Enzyme zurückzuführen ist. - Über die Wirksamkeit spezifischer dsRNA in bezug auf die Hemmung der Genexpression, insbesondere in Säugerzellen und humanen Zellen, ist in diesem Artikel nichts ausgesagt.

Aufgabe der vorliegenden Erfindung ist es, die Nachteile nach dem Stand der Technik zu beseitigen. Es soll insbesondere ein möglichst effizientes Verfahren, Medikament bzw. eine möglichst effiziente Verwendung zur Herstellung eines Medikaments angegeben werden, mit dem/der eine besonders wirksame Hemmung der Expression eines vorgegebenen Zielgens bewirkbar ist.

Diese Aufgabe wird durch die Merkmale der Ansprüche 1, 24 und 48 gelöst. Vorteilhafte Ausgestaltungen ergeben sich aus den Ansprüchen 2 bis 23, 25 bis 47 und 49 bis 72.

Erfindungsgemäß ist zur Hemmung der Expression eines vorgegebenen Zielgens in einer Säugerzelle in vitro vorgesehen, ein 15 bis 49 Basenpaare aufweisendes Oligoribonukleotid mit doppelsträngiger Struktur (dsRNA) in die Säugerzelle einzuführen, wobei ein Strang der dsRNA einen zum Zielgen zumindest abschnittsweise komplementären höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet wird, wobei der durch die Nukleotidpaare bewirkte Zusammenhalt des komplementären Bereichs II durch eine weitere chemisch Verknüpfung erhöht wird, und wobei die chemische Verknüpfung an einem Ende des komplementären Bereichs II hergestellt wird. Das Oligoribonukleotid weist zumindest abschnittsweise eine definierte Nukleotidsequenz auf. Der definierte Abschnitt kann auf den komplementären Bereich I beschränkt sein. Es kann aber auch sein, daß das doppelsträngige Oligoribonukleotid insgesamt eine definierte Nukleotidsequenz aufweist. Die dsRNA kann länger als der zum Zielgen komplementäre Bereich I sein. Der komplementäre Bereich I kann endständig angeordnet oder in die dsRNA eingeschaltet sein. Eine solche dsRNA kann synthetisch bzw. enzymatisch mit gängigen Verfahren hergestellt werden.

Es hat sich überraschenderweise gezeigt, daß bereits bei einer Länge des komplementären Breichs I von höchstens 49 Basenpaaren eine wirksame Hemmung der Expression des Zielgens erreicht werden kann. Entsprechende Oligoribonukleotide können mit geringerem Herstellungsaufwand bereitgestellt werden.

Insbesondere dsRNA mit einer Länge von mehr als 50 Nukleotidpaaren induziert in Säugerzellen und humanen Zellen bestimmte zelluläre Mechanismen, z.B. die dsRNA-abhängige Proteinkinase oder das 2-5A-System. Das führt zum Verschwinden des durch die eine definierte Sequenz aufweisende dsRNA vermittelten Interferenzeffektes. Dadurch wird die Proteinbiosynthese in der Zelle blockiert. Insbesondere dieser Nachteil wird durch die vorliegende Erfindung beseitigt.

Weiterhin ist die Aufnahme von dsRNA mit kurzer Kettenlänge in die Zelle bzw. in den Zellkern gegenüber längerkettigen dsRNAs deutlich erleichtert.

Es hat sich als vorteilhaft erwiesen, daß die dsRNA verpackt in micellare Strukturen, vorzugsweise in Liposomen, vorliegt. Die dsRNA kann gleichfalls in virale natürliche Kapside oder in auf chemischem oder enzymatischem Weg hergestellte künstliche Kapside oder davon abgeleitete Strukturen eingeschlossen sein. - Die vorgenannten Merkmale ermöglichen ein Einschleusen der dsRNA in vorgegebene Zielzellen.

Das zu hemmende Gen wird zweckmäßigerweise in eukaryontischen Zellen exprimiert. Das Zielgen kann ausgewählt sein aus: Onkogen, Cytokin-Gen, Id-Protein-Gen, Entwicklungsgen, Priongen. Es kann auch in pathogenen Organismen, vorzugsweise in Plasmodien, exprimiert werden. Es kann Bestandteil eines, vorzugsweise humanpathogenen, Virus oder Viroids sein. - Das vorgeschlagene Verfahren ermöglicht die Herstellung von Mitteln zur Therapie genetisch gesteuerter Krankheiten, z.B. Krebs, viraler Erkrankungen oder Morbus Alzheimer.

Das Virus oder Viroid kann auch ein tier- oder planzenpathogenes Virus oder Viroid sein. In diesem Fall erlaubt das erfindungsgemäße Verfahren auch die Bereitstellung von Mitteln zur Behandlung von Tier- oder Pflanzenkrankheiten.

Die dsRNA kann abschnittsweise doppelsträngig ausgebildet sein. Die Enden der dsRNA können modifiziert werden, um einem Abbau in der Zelle oder einer Dissoziation in die Einzelstränge entgegenzuwirken. Eine Dissoziation tritt insbesondere bei Verwendung niedriger Konzentrationen oder kurzer Kettenlängen auf. Zur besonders wirksamen Hemmung der Dissoziation kann der durch die Nukleotidpaare bewirkte Zusammenhalt des komplementären Bereichs II durch mindestens eine weitere chemische Verknüpfung erhöht werden. - Eine erfindungsgemäße dsRNA, deren Dissoziation vermindert ist, weist eine höhere Stabilität gegen enzymatischen und chemischen Abbau in der Zelle bzw. im Organismus auf.

Die chemische Verknüpfung wird zweckmäßigerweise durch eine kovalente oder ionische Bindung, eine Wasserstoffbrückenbindung, hydrophobe Wechselwirkungen, vorzugsweise van-der-Waalsoder Stapelungswechselwirkungen, oder durch Metall-Ionenkoordination gebildet. Sie ist erfindungsgemäß an einem, und kann an beiden Ende/n des komplementären Bereichs II hergestellt werden.

Es hat sich weiter als vorteilhaft erwiesen, daß die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind. Die chemische Verknüpfung kann auch durch in den komplementären Bereichen II anstelle von Purinen benutzte Purinanaloga gebildet werden. Von Vorteil ist es ferner, daß die chemische Verknüpfung durch in den komplementären Bereichen II eingeführte Azabenzoleinheiten gebildet wird. Sie kann außerdem durch in den komplementären Bereichen II anstelle von Nukleotiden benutzte verzweigte Nukleotidanaloga gebildet werden.

Es hat sich als zweckmäßig erwiesen, daß zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxylbenzoyl)-cystamin; 4-Thiouracil; Psoralen. Ferner kann die chemische Verknüpfung durch an den Enden des doppelsträngigen Bereichs angebrachte Thiophosphoryl-Gruppen gebildet werden. Vorzugsweise wird die chemische Verknüpfung an den Enden des doppelsträngigen Bereichs durch Tripelhelix-Bindungen hergestellt.

Die chemische Verknüpfung kann zweckmäßigerweise durch ultraviolettes Licht induziert werden.

Die Nukleotide der dsRNA können modifiziert sein. Dies wirkt einer Aktivierung einer von doppelsträngiger RNA abhängigen Proteinkinase, PKR, in der Zelle entgegen. Vorteilhafterweise ist mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt. Mindestens ein Nukleotid in mindestens einem Strang des komplementären Bereichs II kann auch ein sogenanntes "locked nucleotide" mit einem, vorzugsweise durch eine 2'-O, 4'-C-Methylenbrücke, chemisch modifizierten Zuckerring sein. Vorteilhafterweise sind mehrere Nukleotide "locked nucleotides".

Nach einer weiteren besonders vorteilhaften Ausgestaltung ist vorgesehen, daß die dsRNA an mindestens ein von einem Virus stammendes, davon abgeleitetes oder ein synthetisch hergestelltes virales Hüllprotein gebunden, damit assoziiert oder davon umgeben wird. Das Hüllprotein kann vom Polyomavirus abgeleitet sein. Es kann das Hüllprotein das Virus-Protein 1 (VP1) und/oder das Virus-Protein 2 (VP2) des Polyomavirus enthalten. Die Verwendung derartiger Hüllproteine ist z.B. aus der DE 19618 797 A1 bekannt, deren Offenbarungsgehalt hiermit einbezogen wird. - Die vorgenannten Merkmale erleichtert wesentlich das Einführen der dsRNA in die Zelle.

Vorzugsweise ist bei Bildung eines Kapsids oder kapsidartigen Gebildes aus dem Hüllprotein die eine Seite zum Inneren des Kapsids oder kapsidartigen Gebildes gewandt. Das gebildete Konstrukt ist besonders stabil.

Die dsRNA kann zum primären oder prozessierten RNA-Transkript des Zielgens komplementär sein. - Die Zelle kann eine Vertebratenzelle oder eine menschliche Zelle sein.

Es können mindestens zwei voneinander verschiedene dsRNAs in die Zelle eingeführt werden, wobei ein Strang jeder dsRNA zumindest abschnittsweise komplementär zu jeweils einem von mindestens zwei verschiedenen Zielgenen ist. Dadurch ist es möglich gleichzeitig die Expression mindestens zwei verschiedener Zielgene zu hemmen. Um die Expression einer von doppelsträngiger RNA abhängigen Proteinkinase, PKR, in der Zelle zu unterdrücken, ist eines der Zielgene vorteilhafterweise das PKR-Gen. Dadurch kann die PKR-Aktivität in der Zelle wirksam unterdrückt werden.

Nach Maßgabe der Erfindung ist ferner ein Medikament mit mindestens einem 15 bis 49 Basenpaare aufweisenden Oligoribonukleotid mit doppelsträngiger Struktur (dsRNA) zur Hemmung der Expression eines vorgegebenen Zielgens in Säugerzellen vorgesehen, wobei ein Strang der dsRNA einen zum Zielgen zumindest abschnittsweise komplementären höchstens 49 aufeinanderfolgende Nukleotidpaare aufweisenden Bereich I aufweist, und ein innerhalb der doppelsträngigen Struktur komplementärer Bereich II aus zwei separaten RNA-Einzelsträngen gebildet ist, wobei der durch die Nukleotidpaare bewirkte Zusammenhalt des komplementären Bereichs II durch eine weitere chemische Verknüpfung erhöht wird, und wobei die chemische Verknüpfung an einem Ende d s komplementären Bereichs II hergestellt ist.-Es hat sich überraschend gezeigt, daß eine solche dsRNA sich als Medikament zur Hemmung der Expression eines vorgegebenen Gens in Säugerzellen eignet. Die Hemmung wird Im Vergleich zur Verwendung einzelsträngiger Oligoribonukleotide bereits bei Konzentrationen bewirkt, die um mindestens eine Größenordnung niedriger sind. Das erfindungsgemäße Medikament Ist hoch wirksam. Es sind geringere Nebenwirkungen zu erwarten. Es hat sich überraschenderweise gezeigt, daß bereits bei einer Länge des komplementären Bereichs I von höchstens 49 Basenpaaren eine effiziente Hemmung der Expression des Zielgens erreicht werden kann. Entsprechende Oligoribonukleotide können mit geringerem Herstellungsaufwand bereitgestellt werden.

Gegenstand der Erfindung ist ferner eine Verwendung eines 15 bis 49 Basenpaare aufweisenden Oligoribonukleotids mit doppelsträngiger Struktur (dsRNA) zur Herstellung eines Medikaments, wobei ein Strang der dsRNA einen zu einem vorgegebenen Zielgen in Saugerzellen zumindest abschnittsweise komplementären höchstens 49 aufeinanderfolgende Nukleotidpaare aufweisenden Bereich 1 aufweist und ein innerhalb der doppelsträngigen Struktur komplementärer Bereich II aus zwei separaten RNA-Einzelsträngen gebildet ist, wobei der durch die Nukleotidpaare bewirkte Zusammenhalt des komplementären Bereichs II durch eine weitere chemische Verknüpfung erhöht ist, und wobei die chemische Verknüpfung an einem Ende des komplementären Bereichs II hergestellt ist.
- Überraschenderweise eignet sich eine solche dsRNA zur Herstellung eines Medikaments zur Hemmung der Expression eines vorgegebenen Gens. Bei einer Verwendung von dsRNA wird die Hemmung im Vergleich zur Verwendung einzelsträngiger Oligoribonukleotide schon bei um eine Größenordnung geringeren Konzentrationen bewirkt. Die erfindungsgemäße Verwendung ermöglicht also die Herstellung besonders wirksamer Medikamente.

Hinsichtlich vorteilhafter Ausgestaltungen des Medikaments und der Verwendung wird auf die Beschreibung der vorangegangenen Merkmale verwiesen.

Nachfolgend werden anhand der Figuren die Beispiele näher erläutert. Es zeigen:
- Fig. 1: die schematische Darstellung eines Plasmids für die *in vitro*-Transkription mit T7- und SP6-Polymerase,
- Fig. 2: RNA nach Elektrophorese auf einem 8%igen Polyacrylamidgel und Ethidiumbromidfärbung,
- Fig. 3: eine Darstellung radioaktiver RNA-Transkripte nach Elektrophorese auf einem 8%igen Polyacrylamidgel mit 7 M Harnstoff mittels eines "Instant Imagers" und
- Fig. 4 a - e: Texas-Rot- und YFP-Fluoreszenz in murinen Fibroblasten.

### Referenzbeispiel 1:

Die Inhibition der Transkription wurde durch sequenzhomologe dsRNA in einem *in vitro*-Transkiptionssystem mit einem Kernextrakt aus humanen HeLa-Zellen nachgewiesen. Die DNA-Matrize für diesen Versuch war das mittels *Bam*HI linearisierte Plasmid pCMV1200.

### Herstellung der Matrizenplasmide:

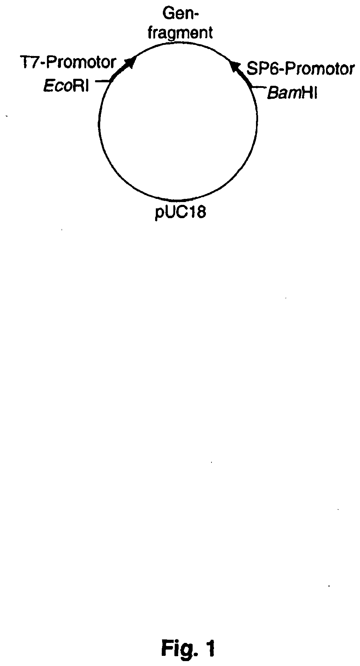

Zur Verwendung bei der enzymatischen Synthese der dsRNA wurde das In Fig. 1 dargestellte Plasmid konstruiert. Dazu wurde zunächst eine Polymerase-Kettenreaktion (PCR) mit der "positive control DNA" des HeLaScribe® Nuclear Extract *in vitro* Transkriptionskits der Firma Promega, Madison, USA als DNA-Matrize durchgeführt. Einer der verwendeten Primer enthielt die Sequenz einer *Eco*RI-Schnittstelle und des T7-RNA-Polymerase-Promotors gemäß Sequenzprotokoll Nr. 1. Der andere Primer enthielt die Sequenz einer *Bam*HI-Schnittstelle und des SP6-RNA-Polymerase-Promotors gemäß Sequenzprotokoll Nr. 2. Darüber hinaus wiesen beide Primer an ihren 3'-Enden identische bzw. komplementäre Bereiche zur DNA-Matrize auf. Die PCR wurde mittels des *"Taq PCR Core Kits"* der Firma Qiagen, Hilden, Deutschland nach Herstellerangaben durchgeführt. In einem Volumen von 100 µl wurden 1,5 mM MgCl₂, je 200 µM dNTP, je 0,5 µM Primer, 2,5 U *Taq*-DNA-Polymerase und etwa 100 ng "positive control DNA" als Matrize in PCR-Puffer eingesetzt. Nach der anfänglichen Denaturierung der Matrizen-DNA durch Erhitzen auf 94°C für 5 Minuten erfolgte die Amplifikation in 30 Zyklen von je 60 Sekunden Denaturierung bei 94°C, 60 Sekunden Annealing bei 5°C unterhalb der berechneten Schmelztemperatur der Primer und 1,5 - 2 Minuten Polymerisation bei 72°C. Nach einer Schlußpolymerisation von 5 Minuten bei 72°C wurden 5 µl des Reaktionsansatzes durch Agarosegelelektrophorese analysiert. Die Länge des so amplifizierten DNA-Fragmentes betrug 400 Basenpaare, wobei 340 Basenpaare der "positive control DNA" entsprachen. Das PCR-Produkt wurde aufgereinigt, mit *Eco*RI und *Bam*HI hydrolysiert und nach erneuter Aufreinigung zur Ligation mit einem ebenfalls durch *Eco*RI und *Bam*HI hydrolysierten pUC18 Vektor eingesetzt. Es erfolgte Transformation von *E. coli* XL1-blue. Das erhaltene Plasmid (pCMV5) trägt ein DNA-Fragment, das am 5'-Ende von dem T7- und am 3'-Ende von dem SP6-Promotor flankiert wird. Durch Linearisierung des Plasmids mit *Bam*HI kann es *in vitro* mit der T7-RNA-Polymerase zur *run*-*off*-Transkription einer 340 Nukleotide langen, in Sequenzprotokoll Nr. 3 dargestellten, einzelsträngigen RNA eingesetzt werden. Wird das Plasmid mit *Eco*RI linearisiert, kann es zur *run-off*-Transkription mit der SP6-RNA-Polymerase eingesetzt werden, wobei der komplementäre Strang entsteht. Entsprechend dem zuvor dargestellten Verfahren wurde auch eine 23 Nukleotide längere RNA synthetisiert. Dazu wurde eine in Sequenzprotokoll Nr. 4 dargestellte DNA über die *Eco*RI und *Bam*HI-Schnittstellen mit dem pUC18 Vektor ligiert.

Als DNA-Matrize für die in vitro-Transkription mit HeLa-Kernextrakt wurde das Plasmid pCMV1200 konstruiert. Dazu wurde ein 1191 bp großes EcoRI/BamHI-Fragment der im HeLaScribe® Nuclear Extract *in vitro* Transkriptionskit enthaltenen Positivkontroll-DNA mittels PCR amplifiziert. Das amplifizierte Fragment umfaßt den 828 bp großen "unmittelbar frühen" CMV-Promotor und ein 363 bp großes transkribierbares DNA-Fragment. Das PCR-Produkt wurde über "T-Überhang"-Ligation mit dem Vektor pGEM-T ligiert. Am 5'-Ende des Fragments ist eine BamHI-Schnittstelle. Das Plasmid wurde durch Hydrolyse mit BamHI linearisiert und als Matrize zur run-off-Transkription eingesetzt.

### In vitro -Transkription der komplementären Einzelstränge:

pCMV5-Plasmid-DNA wurde mit EcoRI bzw. *Bam*HI linearisiert. Sie wurde als DNA-Matrize für eine *in vitro-*Transkription der komplementären RNA-Einzelstränge mit SP6- bzw. T7-RNA-Polymerase verwendet. Dazu wurde das "Riboprobe *in vitro* Transcription" System der Firma Promega, Madison, USA eingesetzt. Nach Herstellerangaben wurden 2 µg linearisierte Plasmid-DNA in 100 µl Transkriptionspuffer und 40 U T7- oder SP6-RNA-Polymerase 5 - 6 Stunden bei 37°C inkubiert. Anschließend wurde die DNA-Matrize durch Zugabe von 2,5 µl RNase-freier DNase RQ1 und Inkubation für 30 Minuten bei 37°C abgebaut. Der Transkriptionsansatz wurde mit H₂O auf 300 µl aufgefüllt und durch Phenolextraktion gereinigt. Die RNA wurde durch Zugabe von 150 µl 7 M Ammoniumacatat und 1125 µl Ethanol gefällt und bis zur Hybridisierung bei -65°C aufbewahrt.

### Herstellung der RNA-Doppelstränge:

Zur Hybridisierung wurden 500 µl der in Ethanol aufbewahrten und gefällten einzelsträngigen RNA abzentrifugiert. Das resultierende Pellet wurde getrocknet und in 30 µl PIPES-Puffer, pH 6,4 in Gegenwart von 80 % Formamid, 400 mM NaCl und 1 mM EDTA aufgenommen. Jeweils 15 µl der komplementären Einzelstränge wurden zusammengegeben und für 10 Minuten auf 85°C erhitzt. Anschließend wurden die Ansätze bei 50°C über Nacht inkubiert und auf Raumtemperatur abgekühlt.

Bei der Hybridisierung wurden nur annähernd äquimolare Mengen der beiden Einzelstränge eingesetzt. Dadurch enthielten die dsRNA-Präparationen einzelsträngige RNA (ssRNA) als Kontamination. Um diese ssRNA-Kontaminationen zu entfernen, wurden die Ansätze nach der Hybridisierung mit den einzelstrangspezifischen Ribonukleasen RNase A aus Rinderpankreas und RNase T1 aus *Aspergillus oryzao* behandelt. RNase A ist eine für Pyrimidine spezifische Endoribonuklease. RNase T1 ist eine Endoribonuklease, die bevorzugt auf der 3'-Seite von Guanosinen schneidet. dsRNA ist kein Substrat für diese Ribonukleasen. Für die RNase-Behandlung wurde zu den Ansätzen in 300 µl Tris, pH 7,4, 300 mM NaCl und 5 mM EDTA 1,2 µl RNaseA in einer Konzentration von 10 mg/ml und 2 µl RNaseT1 in einer Konzentration von 290 µg/ml zugegeben. Die Ansätze wurden 1,5 Stunden bei 30°C inkubiert. Danach wurden die RNasen durch Zugabe von 5 µl Proteinase K in einer Konzentration von 20 mg/ml sowie 10 µl 20%iges SDS und Inkubation für 30 Minuten bei 37°C denaturiert. Die dsRNA wurde durch Phenol-Extraktion gereinigt und mit Ethanol gefällt. Um die Vollständigkeit des RNase-Verdaus überprüfen zu können, wurden zwei Kontrollansätze mit ssRNA analog zu den Hybridisierungsansätzen behandelt.

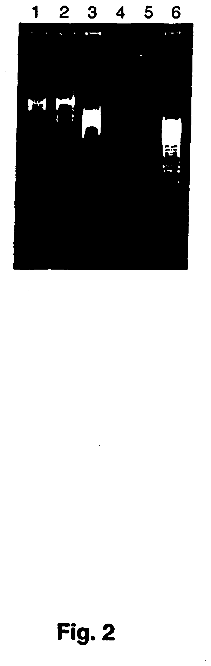

Das getrocknete Pellet wurde in 15 µl TE-Puffer, pH 6,5 aufgenommen und auf einem 8%igen Gel einer nativen Polyacrylamidgelelektrophorese unterzogen. Das Acrylamidgel wurde anschließend in einer Ethidiumbromidlösung gefärbt und in einem Wasserbad gespült. Fig. 2 zeigt die auf einem UV-Transilluminator sichtbar gemachte RNA. Die auf Spur 1 aufgetragene *sense-* und die auf Spur 2 aufgetragene *antisense-*RNA zeigten unter den gewählten Bedingungen ein anderes Laufverhalten als die auf Spur 3 aufgetragene dsRNA des Hybridisierungsansatzes. Die auf den Spuren 4 bzw. 5 aufgetragene RNase-behandelte *sense-* bzw *antisense*-RNA erzeugte keine sichtbare Bande. Dies zeigt, daß die einzelsträngigen RNAs vollständig abgebaut wurden. Die auf Spur 6 aufgetragene RNase-behandelte dsRNA des Hybridisierungsansatzes ist resistent gegenüber der RNase-Behandlung. Die im nativen Gel im Vergleich zu der auf Spur 3 aufgetragenen dsRNA schneller wandernde Bande resultiert aus dsRNA, die frei von ssRNA ist. Neben der dominierenden Hauptbande treten nach der RNase-Behandlung schwächere, schneller wandernde Banden auf.

### in vitro -Transkriptions-Test mit menschlichem Zellkernextrakt:

Unter Verwendung des HeLaScribe® Nuclear Extract *in vitro* Transkriptionskits der Firma Promega, Madison, USA wurde die Transkriptionseffizienz des oben angegebenen, im Plasmid pCMV1200 enthaltenen, zur "positive control DNA" homologen DNA-Fragments in Gegenwart der sequenzhomologen dsRNA (dsRNA-CMV5) bestimmt. Außerdem wurde der Einfluß der nichtsequenzhomologen, dem "Gelb fluoreszierenden Protein" (YFP)-Gen entsprechenden dsRNA (dsRNA-YFP) untersucht. Diese dsRNA war analog zur sequenzhomologen dsRNA hergestellt worden. Die Sequenz eines Stranges dieser dsRNA ist Sequenzprotokoll Nr. 5 zu entnehmen. Als Matrize für die *run-off-*Transkription diente das Plasmid pCMV1200. Es trägt den unmittelbar frühen" Promotor des Cytomegalievirus, der von der eukaryotischen RNA-Polymerase II erkannt wird, und ein transkribierbares DNA-Fragment. Die Transkription erfolgte mittels des HeLa-Kernextrakts, der alle notwendigen Proteine für eine Transkription enthält. Durch Zugabe von [α-³²P]rGTP zum Transkriptionsansatz wurde radioaktiv markiertes Transkript erhalten. Das verwendete [α-³²P]rGTP hatte eine spezifische Aktivität von 400 Ci/mmol, 10 mCi/ml. Pro Ansatz wurden 3 mM MgCl₂, je 400 µM rATP, rCTP, rUTP, 16 µM rGTP, 0,4 µM [α-³²P]rGTP und je nach Versuch 1 fmol linearisierte Plasmid-DNA und verschiedene Mengen an dsRNA in Transkriptionspuffer eingesetzt. Jeder Ansatz wurde mit H₂O auf ein Volumen von 8,5 µl aufgefüllt. Die Ansätze wurden vorsichtig gemischt. Zum Starten der Transkription wurden 4 U HeLa-Kernextrakt in einem Volumen von 4 µl zugegeben und für 60 Minuten bei 30°C inkubiert. Die Reaktion wurde durch Zugabe von 87,5 µl auf 30°C erwärmten Stopp-Mix beendet. Zur Entfernung der Proteine wurden die Ansätze mit 100 µl Phenol/Chloroform/Isoamylalkohol (25:24:1, v/v/v), gesättigt mit TE-Puffer, pH 5,0, versetzt und 1 Minute kräftig gemischt. Zur Phasentrennung wurde etwa 1 Minute bei 12000 rpm zentrifugiert und die obere Phase in ein neues Reaktionsgefäß überführt. Zu jedem Ansatz wurden 250 µl Ethanol zugegeben. Die Ansätze wurden gut gemischt und für mindestens 15 Minuten auf Trockeneis/Methanol inkubiert. Zur Präzipitation der RNA wurden die Ansätze 20 Minuten bei 12000 rpm und 4°C zentrifugiert. Der Überstand wurde verworfen. Das Pellet wurde 15 Minuten im Vakuum getrocknet und in 10 µl H₂O resuspendiert. Zu jedem Ansatz wurden 10 µl denaturierender Probenpuffer zugegeben. Die Trennung des freien GTP vom entstandenen Transkript erfolgte mittels denaturierender Polyacrylamid-Gelelektrophorese auf einem 8%igen Gel mit 7 M Harnstoff. Die bei der Transkription mit HeLa-Kernextrakt gebildeten RNA-Transkripte in denaturierendem Probenpuffer wurden für 10 Minuten auf 90°C erhitzt und 10 µl davon sofort in die frisch gespülten Probentaschen aufgetragen. Die Elektrophorese erfolgte bei 40 mA. Die Menge der bei der Transkription gebildeten radioaktiven ssRNA wurde nach der Elektrophorese mit Hilfe eines *Instant Imager* analysiert*.*

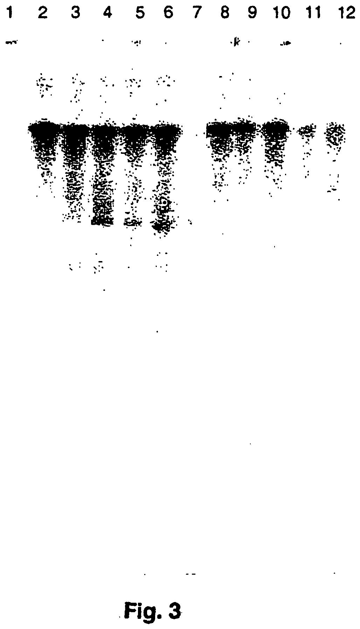

Fig. 3 zeigt die mittels des *Instant Imagers* dargestellte radioaktive RNA aus einem repräsentativen Tests. Es wurden aus folgenden Transkriptionsansätzen gewonne Proben aufgetragen:

| | |
|---|---|
| Spur 1 | ohne Matrizen-DNA, ohne dsRNA; |
| Spur 2 | 50 ng Matrizen-DNA, ohne dsRNA; |
| Spur 3 | 50 ng Matrizen-DNA, 0,5 µg dsRNA-YFP; |
| Spur 4 | 50 ng Matrizen-DNA, 1,5 µg dsRNA-YFP; |
| Spur 5 | 50 ng Matrizen-DNA, 3 µg dsRNA-YFP; |
| Spur 6 | 50 ng Matrizen-DNA, 5 µg dsRNA-YFP; |
| Spur 7 | ohne Matrizen-DNA, 1,5 dsRNA-YFP; |
| Spur 8 | 50 ng Matrizen-DNA, ohne dsRNA; |
| Spur 9 | 50 ng Matrizen-DNA, 0,5 µg dsRNA-CMV5; |
| Spur 10 | 50 ng Matrizen-DNA, 1,5 µg dsRNA-CMV5; |
| Spur 11 | 50 ng Matrizen-DNA, 3 µg dsRNA-CMV5; |
| Spur 12 | 50 ng Matrizen-DNA, 5 µg dsRNA-CMV5; |

Es zeigte sich eine deutliche Verringerung der Menge an Transkript in Gegenwart von sequenzhomologer dsRNA im Vergleich zum Kontrollansatz ohne dsRNA sowie auch zu den Ansätzen mit nicht-sequenzhomologer dsRNA-YFP. Die Positivkontrolle in Spur 2 zeigt, daß bei der *in vitro*-Transkription mit HeLa-Kernextrakt radioaktives Transkript gebildet wurde. Der Ansatz dient zum Vergleich mit den Transkriptionsansätzen, die in Gegenwart von dsRNA inkubiert worden waren. Die Spuren 3 bis 6 zeigen, daß die Zugabe von nicht-sequenzspezifischer dsRNA-YFP keinen Einfluß auf die Menge des gebildeten Transkripts hat. Die Spuren 9 bis 12 zeigen, daß die Zugabe einer zwischen 1,5 und 3 µg liegenden Menge sequenzspezifischer dsRNA-CMV5 zu einer Abnahme der gebildeten Transkript-Menge führt. Um auszuschließen, daß die beobachteten Effekte nicht auf der dsRNA, sondern auf einer möglicherweise bei der Herstellung der dsRNA unabsichtlich mitgeführten Kontamination beruhen, wurde eine weitere Kontrolle durchgeführt. Einzelstrang-RNA wurde wie oben beschrieben transkribiert und anschließend der RNase-Behandlung unterzogen. Mittels nativer Polyacrylamidgelelektrophorese konnte gezeigt werden, daß die ssRNA vollständig abgebaut worden war. Dieser Ansatz wurde wie die Hybridisierungsansätze einer Phenolextraktion und einer Ethanolfällung unterzogen und anschließend in TE-Puffer aufgenommen. Auf diese Weise wurde eine Probe erhalten, die keine RNA enthielt, aber mit den gleichen Enzymen und Puffern behandelt worden war wie die dsRNA. Spur 8 zeigt, daß der Zusatz dieser Probe keinen Einfluß auf die Transkription hatte. Die Abnahme des Transkripts bei Zugabe sequenzspezifischer dsRNA kann deshalb eindeutig der dsRNA selbst zugeschrieben werden. Die Reduzierung der Transkript-Menge eines Gens in Gegenwart von dsRNA bei einem menschlichen Transkriptionssystem zeigt eine Hemmung der Expression des entsprechenden Gens an. Dieser Effekt ist auf einen neuartigen, durch die dsRNA bedingten Mechanismus zurückzuführen.

### Ausführungsbeispiel 2:

Als Testsystem für diese *in vivo*-Experimente diente die murine Fibroblasten-Zellinie NIH3T3, ATCC CRL-1658. Mit Hilfe der Mikroinjektion wurde das YFP-Gen in die Zellkerne eingebracht. Die Expression des YFP wurde unter dem Einfluß gleichzeitig mittransfizierter sequenzhomologer dsRNA untersucht. Diese dsRNA-YFP ist über eine Länge von 315 bp zum 5'-Bereich des YFP-Gens homolog. Die Nukleotidsequenz eines Strangs der dsRNA-YFP ist in Sequenzprotokoll Nr. 5 wiedergegeben. Die Auswertung unter dem Fluoreszenzmikroskop erfolgte 3 Stunden nach Injektion anhand der grün-gelben Fluoreszenz des gebildeten YFP.

### Konstruktion des Matrizenplasmids und Herstellung der dsRNA:

Als Matrize für die Herstellung der YFP-dsRNA mittels T7- und SP6-*in vitro*-Transkription wurde ein Plasmid nach dem gleichen Prinzip wie im Ausführungsbeispiel 1 beschrieben konstruiert. Das gewünschte Genfragment wurde unter Verwendung des Primers *Eco*_T7_YFP gemäß Sequenzprotokoll Nr. 6 und *Bam*_SP6_YFP gemäß Sequenzprotokoll Nr. 7 mittels PCR amplifiziert und analog zu der obigen Beschreibung zur Herstellung der dsRNA verwendet. Die erhaltene dsRNA-YFP ist identisch mit der in Ausführungsbeispiel 1 als nicht-sequenzspezifische Kontrolle verwendeten dsRNA.

Es wurde eine am 3'-Ende der RNA gemäß Sequenzprotokoll Nr. 8 über eine C18-Linkergruppe chemisch mit dem 5'-Ende der komplementären RNA verknüpfte dsRNA (L-dsRNA) hergestellt. Dazu wurden mit Disulfid-Brükken modifizierte Synthone verwendet. Das 3'-terminale Synthon ist über den 3'-Kohlenstoff mit einer aliphatischen Linker-Gruppe über eine Disulfidbrücke an den festen Träger gebunden. Bei dem zum 3'-terminalen Synthon des einen Oligoribonukleotids komplementären 5'-terminalen Synthon des komplementären Oligoribonukleotids ist die 5'-Tritylschutzgruppe über einen weiteren aliphatischen Linker und eine Disulfidbrücke gebunden. Nach Synthese der beiden Einzelstränge, Entfernen der Schutzgruppen und Hybridisierung der komplementären Oligoribonukleotide gelangen die entstehenden Thiolgruppen in räumliche Nachbarschaft zueinander. Durch Oxidation werden die Einzelstränge über ihre aliphatischen Linker und eine Disulfidbrücke miteinander verknüpft. Anschließend erfolgt Reinigung mit Hilfe der HPLC.

### Vorbereitung der Zellkulturen:

Die Zellen wurden in DMEM mit 4,5 g/l Glucose, 10 % fötalem Rinderserum unter 7,5 % CO₂-Atmosphäre bei 37°C in Kulturschalen inkubiert und vor Erreichen der Konfluenz passagiert. Das Ablösen der Zellen erfolgte mit Trypsin/EDTA. Zur Vorbereitung der Mikroinjektion wurden die Zellen in Petrischalen überführt und bis zu Bildung von Mikrokolonien weiter inkubiert.

### Mikroinjektion:

Die Kulturschalen wurde zur Mikroinjektion für ca. 10 Minuten aus dem Inkubator genommen. Es wurde in ca. 50 Zellkerne pro Ansatz innerhalb eines markierten Bereichs unter Verwendung des Mikroinjektionssystems AIS der Firma Carl Zeiss, Göttingen, Deutschland einzeln injiziert. Anschließend wurden die Zellen weitere drei Stunden inkubiert. Für die Mikroinjektion wurden Borosilikat-Glaskapillaren der Firma Hilgenberg GmbH, Malsfeld, Deutschland mit einem Spitzendurchmesser unter 0,5 µm vorbereitet. Die Mikroinjektion wurde mit einem Mikromanipulator der Firma Narishige Scientific Instrument Lab., Tokyo, Japan durchgeführt. Die Injektionsdauer betrug 0,8 Sekunden, der Druck ca. 100 hPa. Für die Transfektion wurde das Plasmid pCDNA-YFP verwendet, das ein ca. 800 bp großes *Bam*HI/*Eco*RI-Fragment mit dem Gen des YFP im Vektor pcDNA3 enthält. Die in die Zellkerne injizierten Proben enthielten 0,01 µg/µl pCDNA-YFP sowie an Dextran-70000 gekoppeltes Texas-Rot in 14 mM NaCl, 3 mM KCl, 10 mM KPO₄, pH 7,5. Zusätzlich wurden ca. 100 pl RNA mit einer Konzentration von 1 µM, bzw. 375 µM im Fall der L-dsRNA, zugegeben.

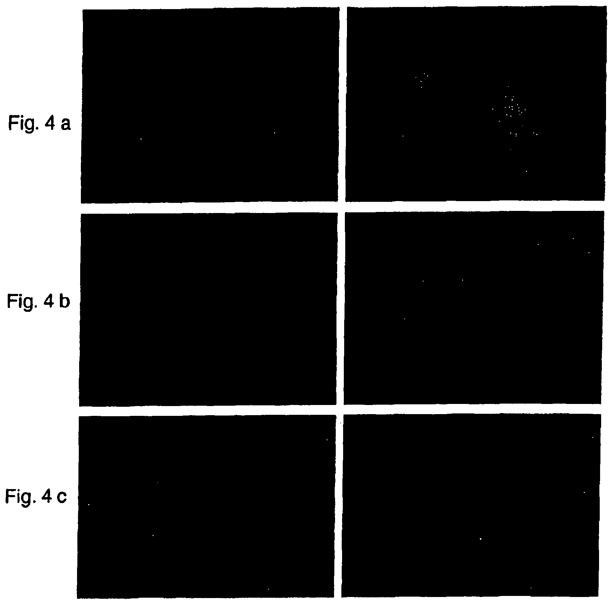

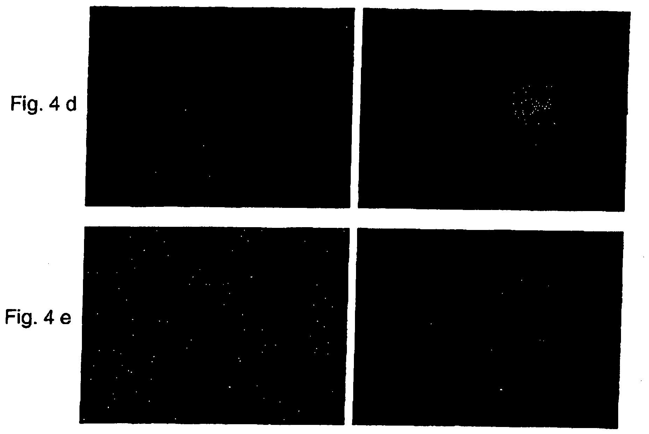

Die Zellen wurden bei Anregung mit Licht der Anregungswellenlänge von Texas-Rot, 568 nm, bzw. von YFP, 488 nm, mittels eines Fluoreszenzmikroskops untersucht. Einzelne Zellen wurden mittels einer digitalen Kamera dokumentiert. Die Figuren 4 a - e zeigen das Ergebnis für NIH3T3-Zellen. Bei den in Fig. 4 a gezeigten Zellen ist *sense*-YFP-ssRNA, in Fig. 4b *antisense*-YFP-ssRNA, in Fig. 4 c dsRNA-YFP, in Fig. 4 d keine RNA und in Fig. 4 e L-dsRNA injiziert worden.

Das jeweils linke Feld zeigt die Fluoreszenz von Zellen, die mit 568 nm angeregt wurden. Rechts ist die Fluoreszenz derselben Zellen bei Anregung mit 488 nm zu sehen. Die Texas-Rot-Fluoreszenz aller dargestellten Zellen zeigt, daß die Injektionslösung erfolgreich in die Zellkerne appliziert wurde und getroffene Zellen nach drei Stunden noch lebendig waren. Abgestorbene Zellen zeigten keine Texas-Rot-Fluoreszenz mehr.

Die jeweils rechten Felder der Figuren 4 a und 4 b zeigen, daß die Expression des YFP bei Injektion der einzelsträngigen RNA in die Zellkerne nicht sichtbar inhibiert wurde. Das rechte Feld der Fig. 4c zeigt Zellen, deren YFP-Fluoreszenz nach Injektion von dsRNA-YFP nicht mehr nachweisbar war. Fig. 4d zeigt als Kontrolle Zellen, in die keine RNA injiziert worden war. Die in Fig. 4 e dargestellte Zelle zeigt durch die Injektion der L-dsRNA, die zum YFP-Gen sequenzhomologe Bereiche aufweist, eine nicht mehr nachweisbare YFP-Fluoreszenz. Dieses Ergebnis belegt, daß auch kürzere dsRNAs zur spezifischen Inhibition der Genexpression bei Säugern verwendet werden können, wenn die Doppelstränge durch chemische Verknüpfung der Einzelstränge stabilisiert werden.

### Literatur:

Asanuma, H., Ito, T., Yoshida, T., Liang, X. & Komiyama, M. (1999). Photoregulation der Bildung und Dissoziation eines DNA-Duplexes durch cis-trans-Isomerisierung einer Azobenzoleinheit. Angew. Chem. 111, 2547-2549.
Azhayeva, E., Azhayev, A., Auriola, S., Tengvall, U., Urtti, A. & Lönnberg, H. (1997). Inhibitory properties of double helix forming circular oligonucleotides. Nucl. Acids Res. 25, 4954-4961.
Castelli, J., Wood, K.A. & Youle, R.J. (1998). The 2-5A system in viral infection and apoptosis. Biomed. Pharmacother. 52, 386-390.
Dolinnaya, N.G., Blumenfeld, M., Merenkova, I., Oretskaya, T.S., Krynetskaya, N.F., Ivanovskaya, M.G., Vasseur, M. & Shabarova, Z.A. (1993). Oligonucleotide circularization by template-directed chemical ligation. Nucl. Acids Res. 21, 5403-5407.
Expert-Bezancon, A., Milet, M. & Carbon, P. (1983). Precise localization of several covalent RNA-RNA cross-link in Escherichia coli 16S RNA. Eur. J. Biochem. 136, 267-274.
Fire, A., Xu, S., Montgomery, M.K., Kostas, S.A., Driver, S.E. & Mello, C.C. (1998). Potent and specific genetic interference by double-stranded RNA in Caenorhabditis elegans. Nature 391, 806-811.
Gao, H., Yang, M., Patel, R. & Cook, A.F. (1995). Circulaization of oligonucleotides by disulfide bridge formation. Nucl. Acids Res. 23, 2025-2029.
Gryaznov, S.M. & Letsinger, R.L. (1993). Template controlled coupling and recombination of oligonucleotide blocks containing thiophosphoryl groups. Nucl. Acids Res. 21, 1403-1408.
Kaufman, R.J. (1999). Double-stranded RNA-activated protein kinase mediates virus-induced apoptosis: A new role for an old actor. Proc. Natl. Acad. Sci. USA 96, 11693-11695.
Lipson, S.E. & Hearst, J.E. (1988). Psoralen cross-linking of ribosomal RNA. In Methods in Enzymology Anonymous pp. 330-341.
Liu, Z.R., Sargueil, B. & Smith, C.W. (1998). Detection of a novel ATP-dependent cross-linked protein at the 5' splice site-U1 small nuclear RNA duplex by methylene bluemediated photo-cross-linking. Mol. Cell. Biol. 18, 6910-6920.
Micura, R. (1999). Cyclic oligoribonucleotides (RNA) by solidphase synthesis. Chem. Eur. J. 5, 2077-2082.
Skripkin, E., Isel, C., Marquet, R., Ehresmann, B. & Ehresmann, C. (1996). Psoralen crosslinking between human immunodeficiency virus type 1 RNA and primer tRNA3Lys. Nucl. Acids Res. 24, 509-514.
Wang, S. & Kool, E.T. (1994). Circular RNA oligonucleotides. Synthesis, nucleic acid binding properties, and a comparison with circular DNAs. Nucl. Acids Res. 22, 2326-2333.
Wang, Z. & Rana, T.M. (1996). RNA conformation in the Tat-TAR complex determined by site-specific photo-cross-linking. Biochem. 35, 6491-6499.
Watkins, K.P. & Agabian, N. (1991). In vivo UV cross-linking of U snRNAs that paticipate in trypanosome transsplicing. Genes & Development 5, 1859-1869.
Wengel, J. (1999). Synthesis of 3'-C- and 4'-C-branched oligodeoxynucleotides and the development of locked nucleic acid (LNA). Acc. Chem. Res. 32, 301-310.
Zwieb, C., Ross, A., Rinke, J., Meinke, M. & Brimacombe, R. (1978). Evidence for RNA-RNA cross-link formation in Escherichia coli ribosomes. Nucl. Acids Res. 5, 2705-2720.

## Patentansprüche

1. Verfahren zur Hemmung der Expression eines vorgegebenen Zielgens in einer Säugerzelle in vitro, wobei ein 15 bis 49 Basenpaare aufweisendes Oligoribonukleotid mit doppelsträngiger Struktur (dsRNA) in die Säugerzelle eingeführt wird, wobei ein Strang der dsRNA einen zum Zielgen zumindest abschnittsweise komplementären höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet wird, wobei der durch die Nukleotidpaare bewirkte Zusammenhalt des komplementären Bereichs II durch eine weitere chemische Verknüpfung erhöht wird, und wobei die chemische Verknüpfung an einem Ende des komplementären Bereichs II hergestellt wird.

2. Verfahren nach Anspruch 1, wobei die dsRNA in micellare Strukturen, vorzugsweise in Liposomen, eingeschlossen wird.

3. Verfahren nach einem der vorhergehenden Ansprüche, wobei das Zielgen in eukaryontischen Zellen exprimiert wird.

4. Verfahren nach einem der vorhergehenden Ansprüche, wobei das Zielgen ausgewählt ist aus: Onkogen, Cytokin-Gen, Id-Protein-Gen, Entwicklungsgen, Priongen.

5. Verfahren nach einem der vorhergehenden Ansprüche, wobei das Zielgen Bestandteil eines Virus oder Viroids ist.

6. Verfahren nach Anspruch 5, wobei das Virus ein humanpathogenes Virus oder Viroid ist.

7. Verfahren nach Anspruch 5, wobei das Virus oder Viroid ein tierpathogenes Virus oder Viroid ist.

8. Verfahren nach einem der vorhergehenden Ansprüche, wobei die Enden der dsRNA modifiziert werden, um einem Abbau in der Zelle oder einer Dissoziation in die Einzelstränge entgegenzuwirken.

9. Verfahren nach einem der vorhergehenden Ansprüche, wobei der durch die Nukleotidpaare bewirkte Zusammenhalt des komplementären Bereichs II durch eine weitere chemische Verknüpfung erhöht wird.

10. Verfahren nach einem der vorhergehenden Ansprüche, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

11. Verfahren nach einem der vorhergehenden Ansprüche, wobei die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind.

12. Verfahren nach einem der vorhergehenden Ansprüche, wobei die chemische Verknüpfung durch in den komplementären Bereichen II anstelle von Purinen benutzten Purinanaloga gebildet wird.

13. Verfahren nach einem der vorhergehenden Ansprüche, wobei die chemische Verknüpfung durch in den komplementären Bereichen II eingeführte Azabenzoleinheiten gebildet wird.

14. Verfahren nach einem der vorhergehenden Ansprüche, wobei die chemische Verknüpfung durch in den komplementären Bereichen II anstelle von Nukleotiden benutzte verzweigte Nukleotidanaloga gebildet wird.

15. Verfahren nach einem der vorhergehenden Ansprüche, wobei zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.

16. Verfahren nach einem der vorhergehenden Ansprüche, wobei die chemische Verknüpfung durch an den Enden des doppelsträngigen Bereichs angebrachte Thiophosphoryl-Gruppen gebildet wird.

17. Verfahren nach einem der vorhergehenden Ansprüche, wobei mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt ist.

18. Verfahren nach einem der vorhergehenden Ansprüche, wobei mindestens ein Nukleotid in mindestens einem Strang des komplementären Bereichs II ein "locked nucleotide" mit einem, vorzugsweise durch eine 2'-O, 4'-C-Methylenbrücke, chemisch modifizierten Zuckerring ist.

19. Verfahren nach einem der vorhergehenden Ansprüche, wobei die dsRNA an mindestens ein von einem Virus stammendes, davon abgeleitetes oder ein synthetisch hergestelltes virales Hüllprotein gebunden, damit assoziiert oder davon umgeben wird.

20. Verfahren nach einem der vorhergehenden Ansprüche, wobei die dsRNA zum primären oder prozessierten RNA-Transkript des Zielgens komplementär ist.

21. Verfahren nach einem der vorhergehenden Ansprüche, wobei die Zelle eine menschliche Zelle ist.

22. Verfahren nach einem der vorhergehenden Ansprüche, wobei mindestens zwei voneinander verschiedene dsRNAs in die Zelle eingeführt werden, wobei ein Strang jeder dsRNA zumindest abschnittsweise komplementär zu jeweils einem von mindestens zwei verschiedenen Zielgenen Ist.

23. Verfahren nach einem der vorhergehenden Ansprüche, wobei eines der Zielgene das PKR-Gen ist.

24. Medikament mit mindestens einem 15 bis 49 Basenpaare aufweisenden Oligoribonukleotid mit doppelsträngiger Struktur (dsRNA) zur Hemmung der Expression eines vorgegebenen Zielgens in Säugerzellen, wobei ein Strang der dsRNA einen zum Zielgen zumindest abschnittsweise komplementären höchstens 49 aufeinanderfolgende Nukleotidpaare aufweisenden Bereich I aufweist und ein innerhalb der doppelstrangigen Struktur komplementärer Bereich II aus zwei separaten RNA-Einzelsträngen gebildet ist, wobei der durch die Nukleotidpaare bewirkte Zusammenhalt des komplementären Bereichs II durch eine weitere chemische Verknüpfung erhöht wird, und wobei die chemische Verknüpfung an einem Ende des komplementären Bereichs II hergestellt ist.

25. Medikament nach Anspruch 24, wobei die dsRNA verpackt in micellare Strukturen, vorzugsweise in Liposomen, vorliegt.

26. Medikament nach einem der Ansprüche 24 oder 25, wobei das Zielgen in eukaryontischen Zellen exprimierbar ist.

27. Medikament nach einem der Ansprüche 24 bis 26, wobei das Zielgen ausgewählt ist aus: Onkogen, Cytokin-Gen, Id-Protein-Gen, Entwicklungsgen, Priongen.

28. Medikament nach einem der Ansprüche 24 bis 27, wobei das Zielgen in pathogenen Organismen, vorzugsweise in Plasmodien, exprimierbar ist.

29. Medikament nach einem der Ansprüche 24 bis 28, wobei das Zielgen Bestandteil eines Virus oder Viroids ist.

30. Medikament nach Anspruch 29, wobei das Virus ein humanpathogenes Virus oder Viroid ist.

31. Medikament nach Anspruch 29, wobei das Virus oder Viroid ein tierpathogenes Virus oder Viroid ist.

32. Medikament nach einem der Ansprüche 24 bis 31, wobei die Enden der dsRNA modifiziert sind, um einem Abbau in der Zelle oder einer Dissoziation in die Einzelstränge entgegenzuwirken.

33. Medikament nach einem der Ansprüche 24 bis 32, wobei der durch die Nukleotidpaare bewirkte Zusammenhalt des komplementären Bereichs II durch eine weitere chemische Verknüpfung erhöht ist.

34. Medikament nach einem der Ansprüche 24 bis 33, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metalllonenkoordination gebildet ist.

35. Medikament nach einem der Ansprüche 24 bis 34, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)- und/oder Polyethylenglycol-Ketten sind.

36. Medikament nach einem der Ansprüche 24 bis 35, wobei die chemische Verknüpfung durch in den komplementären Bereichen II anstelle von Purinen benutzte Purinanaloga gebildet ist.

37. Medikament nach einem der Ansprüche 24 bis 36, wobei die chemische Verknüpfung durch in die komplementären Bereiche II eingeschaltete Azabenzoleinheiten gebildet ist.

38. Medikament nach einem der Ansprüche 24 bis 37, wobei die chemische Verknüpfung durch in den komplementären Bereichen II anstelle von Nukleotiden benutzte verzweigte Nukleotidanaloga gebildet ist.

39. Medikament nach einem der Ansprüche 24 bis 38, wobei zur Herstellung der chemischen Verknüpfung mindestenes eine der folgenden Gruppen benutzt wird: Methylenblau; bifunktionelle Gruppen, vorzugsweise Bis-(2-chlorethyl)-amin; N-acetyl-N'-(p-glyoxyl-benzöyl)-cystamin; 4-Thiouracil; Psoralen.

40. Medikament nach einem der Ansprüche 24 bis 39, wobei die chemische Verknüpfung durch an den Enden des doppelsträngigen Bereichs vorgesehene Thiophosphoryl-Gruppen gebildet ist.

41. Medikament nach einem der Ansprüche 24 bis 40, wobei mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt ist.

42. Medikament nach einem der Ansprüche 24 bis 41, wobei mindestens ein Nukleotid in mindestens einem Strang des komplementären Bereichs II ein "locked nucleotide" mit einem, vorzugsweise durch eine 2'-O, 4'-C-Methylenbrücke, chemisch modifizierten Zuckerring ist.

43. Medikament nach einem der Ansprüche 24 bis 42, wobei die dsRNA an mindestens ein von einem Virus stammendes, davon abgeleitetes oder ein synthetisch hergestelltes virales Hüllprotein gebunden, damit assoziiert oder davon umgeben ist.

44. Medikament nach einem der Ansprüche 24 bis 43, wobei die dsRNA zum primären oder prozessierten RNA-Transkript des Zielgens komplementär ist.

45. Medikament nach einem der Ansprüche 24 bis 44, wobei die Zelle eine menschliche Zelle ist.

46. Medikament nach einem der Ansprüche 24 bis 45, wobei darin mindestens zwei voneinander verschiedene dsRNAs enthalten sind, wobei ein Strang jeder dsRNA zumindest abschnittsweise komplementär zu jeweils einem von mindestens zwei verschiedenen Zielgenen ist.

47. Medikament nach Anspruch 46, wobei eines der Zielgene das PKR-Gen ist.

48. Verwendung eines 15 bis 49 Basenpaare aufweisenden Oligoribonukleotids mit doppelsträngiger Struktur (dsRNA) zur Herstellung eines Medikaments, wobei ein Strang der dsRNA einen zu einem vorgegebenen Zielgen in Säugerzellen zumindest abschnittsweise komplementären höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet ist, wobei der durch die Nukleotidpaare bewirkte Zusammenhalt des komplementären Bereichs II durch eine weitere chemische Verknüpfung erhöht ist, und wobei die chemische Verknüpfung an einem Ende des komplementären Bereichs II hergestellt ist.

49. Verwendung nach Anspruch 48, wobei die dsRNA verpackt in micellare Strukturen, vorzugsweise in Liposomen, vorliegt.

50. Verwendung nach einem der Ansprüche 48 oder 49, wobei das Zielgen in eukaryontischen Zellen exprimierbar ist.

51. Verwendung nach einem der Ansprüche 48 bis 50, wobei das Zielgen ausgewählt ist aus: Onkogen, Cytokin-Gen, Id-Protein-Gen, Entwicklungsgen, Priongen.

52. Verwendung nach einem der Ansprüche 48 bis 51, wobei das Zielgen in pathogenen Organismen, vorzugsweise in Plasmodien, exprimierbar ist.

53. Verwendung nach einem der Ansprüche 48 bis 52, wobei das Zielgen Bestandteil eines Virus oder Viroids ist.

54. Verwendung nach Anspruch 53, wobei das Virus ein humanpathogenes Virus oder Viroid ist.

55. Verwendung nach Anspruch 53, wobei das Virus oder Viroid ein tierpathogenes Virus oder Viroid ist.

56. Verwendung nach einem der Ansprüche 48 bis 55, wobei die Enden der dsRNA modifiziert sind, um einem Abbau in der Zelle oder einer Dissoziation in die Einzelstränge entgegenzuwirken.

57. Verwendung nach einem der Ansprüche 48 bis 56, wobei der durch die Nukleotidpaare bewirkte Zusammenhalt des komplementären Bereichs II durch eine, weitere chemische Verknüpfung erhöht ist.

58. Verwendung nach einem der Ansprüche 48 bis 57, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionerikoordination gebildet ist.

59. Verwendung nach einem der Ansprüche 48 bis 58, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)- und/oder Polyethylenglycol-Ketten sind.

60. Verwendung nach einem der Ansprüche 48 bis 59, wobei die chemische Verknüpfung durch in den komplementären Bereichen II anstelle von Purinen benutzte Purinanaloga gebildet ist.

61. Verwendung nach einem der Ansprüche 48 bis 60, wobei die chemische Verknüpfung durch in den komplementären Bereichen II eingeführte Azabenzoleinheiten gebildet ist.

62. Verwendung nach einem der Ansprüche 48 bis 61, wobei die chemische Verknüpfung durch in den komplementären Bereichen II anstelle von Nukleotiden benutzte verzweigte Nukleotidanaloga gebildet ist.

63. Verwendung nach einem der Ansprüche 48 bis 62, wobei zur Herstellung der chemischen Verknüpfung mindestenes eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.

64. Verwendung nach einem der Ansprüche 48 bis 63, wobei die chemische Verknüpfung durch an den Enden des doppelsträngigen Bereichs angebrachte Thiophosphoryl-Gruppen gebildet ist.

65. Verwendung nach einem der Ansprüche 48 bis 64, wobei mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt ist.

66. Verwendung nach einem der Ansprüche 48 bis 65, wobei mindestens ein Nukleotid in mindestens einem Strang des komplementären Bereichs II ein "locked nucleotide" mit einem, vorzugsweise durch eine 2'-O, 4'-C-Methylenbrücke, chemisch modifizierten Zuckerring ist.

67. Verwendung nach einem der Ansprüche 48 bis 66, wobei die dsRNA an mindestens ein von einem Virus stammendes, davon abgeleitetes oder ein synthetisch hergestelltes virales Hüllprotein gebunden, damit assoziiert oder davon umgeben ist.

68. Verwendung nach einem der Ansprüche 48 bis 67, wobei die dsRNA zum primären oder prozessierten RNA-Transkript des Zielgens komplementär ist.

69. Verwendung nach einem der Ansprüche 48 bis 68, wobei die Zelle eine menschliche Zelle ist.

70. Verwendung nach einem der Ansprüche 48 bis 69, wobei mindestens zwei voneinander verschiedene dsRNAs verwendet werden, wobei ein Strang jeder dsRNA zumindest abschnittsweise komplementär zu jeweils einem von mindestens zwei verschiedenen Zielgenen ist.

71. Verwendung nach Anspruch 70, wobei eines der Zielgene das PKR-Gen ist.

72. Verwendung nach einem der Ansprüche 48 bis 71, wobei das Medikament in die Blutbahn oder das Interstitium des zu therapierenden Organismus injizierbar ist.

## Claims

1. A method for inhibiting the expression of a given target gene in a cell of a mammal in vitro, wherein an oligoribonucleotide of double-stranded structure (dsRNA) having 15 to 49 base pairs is introduced into the mammalian cell, wherein one strand of the dsRNA has a region I which is complementary, at least in segments, to the target gene, wherein the complementary region has 49 consecutive nucleotide pairs at the most, and a region II, which is complementary within the double-stranded structure, is formed from two separate RNA single strands, and wherein the cohesion of the complementary region II, which is achieved by the nucleotide pairs, is increased by a further chemical linkage, and wherein the chemical linkage is produced at one end of the complementary region II.

2. The method according to claim 1, wherein the dsRNA is enclosed in micellar structures, preferably in liposomes.

3. The method according to any one of the preceding claims, wherein the target gene is expressed in eukaryotic cells.

4. The method according to any one of the preceding claims, wherein the target gene is selected from the following group: oncogene, cytokine gene, Id-protein gene, development gene, prion gene.

5. The method according to any one of the preceding claims, wherein the target gene is part of a virus or a viroid.

6. The method according to claim 5, wherein the virus is a virus or viroid pathogenic for humans.

7. The method according to claim 5, wherein the virus or viroid is a virus or viroid pathogenic for animals.

8. The method according to any one of the preceding claims, wherein the ends of the dsRNA are modified in order to counteract degradation in the cell or dissociation into the single strands.

9. The method according to any one of the preceding claims, wherein the cohesion of the complementary region II achieved by the nucleotide pairs is increased by a further chemical linkage.

10. The method according to any one of the preceding claims, wherein the chemical linkage is formed by a covalent or ionic bond, a hydrogen bond, hydrophobic interactions, preferably van-der-Waals or stacking interactions, or by metal-ion coordination.

11. The method according to any one of the preceding claims, wherein the chemical linkage is formed by one or more linkage groups, wherein preferably the linkage groups are poly-(oxyphosphinicooxy 1,3-propandiol) and/or polyethylene glycol chains.

12. The method according to any one of the preceding claims, wherein the chemical linkage is formed by purine analogues used in the complementary regions II in place of purines.

13. The method according to any one of the preceding claims, wherein the chemical linkage is formed by azabenzene units introduced into the complementary regions II.

14. The method according to any one of the preceding claims, wherein the chemical linkage is formed by branched nucleotide analogues used in the complementary regions II in place of nucleotides.

15. The method according to any one of the preceding claims, wherein at least one of the following groups is used for generating the chemical linkage: methylene blue; bifunctional groups, preferably bis-(2-chloroethyl)amine; N-acetyl-N'(p-glyoxylbenzoyl)-cystamine; 4-thiouracil; psoralene.

16. The method according to any one of the preceding claims, wherein the chemical linkage is formed by thiophosphoryl groups attached at the ends of the double-stranded structure.

17. The method according to any one of the preceding claims, wherein at least one 2'-hydroxyl group of the nucleotides of the dsRNA in the complementary region II is replaced by a chemical group, preferably a 2'-amino or a 2'-methyl group.

18. The method according to any one of the preceding claims, wherein at least one nucleotide in at least one strand of the complementary region II is a "locked nucleotide" with a sugar ring which is chemically modified, preferably by a 2'-O, 4'-C-methylene bridge.

19. The method according to any one of the preceding claims, wherein the dsRNA is bound to, associated with or surrounded by, at least one viral coat protein which originates from a virus, is derived therefrom or has been prepared synthetically.

20. The method according to any one of the preceding claims, wherein one strand of the dsRNA is complementary to the primary or processed RNA transcript of the target gene.

21. The method according to any one of the preceding claims, wherein the cell is a human cell.

22. The method according to any one of the preceding claims, wherein at least two dsRNAs which differ from each other are introduced into the cell, wherein at least segments of one strand of each dsRNA are complementary to one of at least two different target cells.

23. The method according to any one of the preceding claims, wherein one of the target genes is the PKR gene.

24. A medicament for inhibiting the expression of a given target gene in cells of a mammal, said medicament comprising at least one oligoribonucleotide of double-stranded structure (dsRNA) having 15 to 49 base pairs, wherein one strand of the dsRNA has a region I which is complementary, at least in segments, to the target gene, wherein the complementary region has 49 consecutive nucleotide pairs at the most, and a region II, which is complementary within the double-stranded structure, is formed from two separate RNA single strands, and wherein the cohesion of the complementary region II, which is achieved by the nucleotide pairs, is increased by a further chemical linkage, and wherein the chemical linkage is produced at one end of the complementary region II.

25. The medicament according to claim 24, wherein the dsRNA is enclosed by micellar structures, preferably liposomes.

26. The medicament according to any one of claims 24 or 25, wherein the target gene can be expressed in eukaryotic cells.

27. The medicament according to any one of claims 24 to 26, wherein the target gene is selected from the following group: oncogene, cytokine gene, Id-protein gene, development gene, prion gene.

28. The medicament according to any one of claims 24 to 27, wherein the target gene can be expressed in pathogenic organisms, preferably in plasmodia.

29. The medicament according to any one of claims 24 to 28, wherein the target gene is part of a virus or a viroid.

30. The medicament according to claim 29, wherein the virus is a virus or viroid pathogenic for humans.

31. The medicament according to claim 29, wherein the virus or viroid is a virus or viroid pathogenic for animals.

32. The medicament according to any one of claims 24 to 31, wherein the ends of the dsRNA are modified in order to counteract degradation in the cell or dissociation into the single strands.

33. The medicament according to any one of claims 24 to 32, wherein the cohesion of the complementary region II achieved by the nucleotide pairs is increased by a further chemical linkage.

34. The medicament according to any one of claims 24 to 33, wherein the chemical linkage is formed by a covalent or ionic bond, a hydrogen bond, hydrophobic interactions, preferably van-der-Waals or stacking interactions, or by metal ion coordination.

35. The medicament according to any one of claims 24 to 34, wherein the chemical linkage is formed by one or more linkage groups, wherein preferably the linkage groups are poly-(oxyphosphinicooxy-1,3-propandiol) and/or polyethylene glycol chains.

36. The medicament according to any one of claims 24 to 35, wherein the chemical linkage is formed by purine analogues used in the complementary regions II in place of purines.

37. The medicament according to any one of claims 24 to 36, wherein the chemical linkage is formed by means of azabenzene units introduced into the complementary regions II.

38. The medicament according to any one of claims 24 to 37, wherein the chemical linkage is formed by means of branched nucleotides analogues used in the complementary regions II in place of nucleotides.

39. The medicament according to any one of claims 24 to 38, wherein at least one of the following groups is used for generating the chemical linkage: methylene blue; bifunctional groups, preferably bis-(2-chlorethyl)amine; N-acetyl-N'-(p-glyoxyl-benzoyl)-cystamine; 4-thiouracil; psoralene.

40. The medicament according to any one of claims 24 to 39, wherein the chemical linkage is formed by means of thiophosphoryl groups provided at the ends of the double-stranded structure.

41. The medicament according to any one of claims 24 to 40, wherein at least one 2'-hydroxyl group of the nucleotides of the dsRNA in the complementary region II is replaced by a chemical group, preferably by a 2'-amino or a 2'-methyl group.

42. The medicament according to any one of claims 24 to 41, wherein at least one nucleotide in at least one strand of the complementary region II is a "locked nucleotide" with a sugar ring which is chemically modified, preferably by a 2'-O, 4'-C-methylene bridge.

43. The medicament according to any one of claims 24 to 42, wherein the dsRNA is linked to, associated with or surrounded by at least one viral coat protein, which originates from a virus, is derived therefrom or has been prepared synthetically.

44. The medicament of any one of claims 24 to 43, wherein the dsRNA is complementary to the primary or the processed RNA transcript of the target gene.

45. The medicament of any one of claims 24 to 44, wherein the cell is a human cell.

46. The medicament of any one of claims 24 to 45 containing at least two dsRNAs differing from one another, wherein at least segments of one strand of each dsRNA are complementary to one of at least two different target genes.

47. The medicament of claim 46, wherein one of the target genes is the PKR gene.

48. Use of an oligoribonucleotide of double-stranded structure (dsRNA) having 15 to 49 base pairs for the preparation of a medicament, wherein one strand of the dsRNA has a region I which is complementary, at least in segments, to a given target gene in mammalian cells, wherein the complementary region has 49 consecutive nucleotide pairs at the most, and a region II, which is complementary within the double-stranded structure, is formed from two separate RNA single strands, and wherein the cohesion of the complementary region II, which is achieved by the nucleotide pairs, is increased by a further chemical linkage, and wherein the chemical linkage is produced at one end of the complementary region II.

49. The use according to claim 48, wherein the dsRNA is enclosed in micellar structures, preferably in liposomes.

50. The use according to any one of claims 48 or 49, wherein the target gene can be expressed in eukaryotic cells.

51. The use according to any one of claims 48 to 50, wherein the target gene is selected from the following group: onkogene, cytokine gene, Id-protein gene, development gene, prion gene.

52. The use according to any one of claims 48 to 51, wherein the target gene can be expressed in pathogenic organisms, preferably in plasmodia.

53. The use according to any one of claims 48 to 52, wherein the target gene is part of a virus or a viroid.

54. The use according to claim 53, wherein the virus is a virus or viroid pathogenic for humans.

55. The use according to claim 53, wherein the virus or viroid is a virus or viroid pathogenic for animals.

56. The use according to any one of claims 48 to 55, wherein the ends of the dsRNA are modified in order to counteract the degradation in the cell or dissociation into the single strands.

57. The use according to any one of claims 48 to 56, wherein the cohesion of the complementary region II achieved by the nucleotide pairs is increased by a further chemical linkage.

58. The use according to any one of claims 48 to 57, wherein the chemical linkage is formed by a covalent or ionic bond, a hydrogen bond, hydrophobic interactions, preferably van-der-Waals or stacking interactions or by metal ion coordination.

59. The use according to any one of claims 48 to 58, wherein the chemical linkage is formed by means of one or more linkage groups, wherein the linkage groups preferably are poly-(oxyphosphinicooxy-1,3-propandiol) and/or polyethylene glycol chains.

60. The use according to any one of claims 48 to 59, wherein the chemical linkage is formed by means of purine analogues used in the complementary regions II in place of purines.

61. The use according to any one of claims 48 to 60, wherein the chemical linkage is formed by the azabenzene units introduced into the complementary regions II.

62. The use according to any one of claims 48 to 61, wherein the chemical linkage is formed by the branched nucleotide analogues used in the complementary regions II in place of nucleotides.

63. The use according to any one of claims 48 to 62, wherein at least one of the following groups is used for generating the chemical linkage: methylene blue; bi-functional groups, preferably bis-(2-chlorethyl)amine; N-acetyl-N'-(p-glyoxyl-benzoyl)-cystamine; 4-thiouracyl; psoralene.

64. The use according to any one of claims 48 to 63, wherein the chemical linkage is formed by thiophosphoryl groups attached to the ends of the double-stranded structure.

65. The use according to any one of claims 48 to 64, wherein at least one 2'-hydroxyl group of the nucleotides of the dsRNA in the complementary region II is replaced by a chemical group, preferably a 2'-amino or a 2'-methyl group.

66. The use according to any one of claims 48 to 65, wherein at least one nucleotide in at least one strand of the complementary region II is a "locked nucleotide" with a sugar ring which is chemically modified, preferably by a 2'-O, 4'-C-methylene bridge.

67. The use according to any one of claims 48 to 66, wherein the dsRNA is linked to, associated with or surrounded by at least one viral coat protein which originates from a virus, is derived therefrom or has been prepared synthetically.

68. The use according to any one of claims 48 to 67, wherein the dsRNA is complementary to the primary or the processed RNA transcript of the target gene.

69. The use according to any one of claims 48 to 68, wherein the cell is a human cell.

70. The use according to any one of claims 48 to 69, wherein at least two dsRNAs differing from one another are used, wherein at least segments of one strand of each dsRNA are complementary to one of at least two different target genes.

71. The use according to claim 70, wherein one of the target genes is the PKR gene.

72. The use according to any one of claims 48 to 71, wherein the medicament can be injected into the blood stream or the interstitium of the organism to undergo therapy.

## Revendications

1. Procédé d'inhibition de l'expression d'un gène cible donné dans une cellule de mammifères in vitro, dans lequel un oligoribonucléotide d'une structure double brin (ARNdb) présentant 15 à 49 paires de base est introduit dans la cellule de mammifères, un brin de l'ARNdb présentant une région I complémentaire au moins sur certains segments du gène cible et qui présente au plus 49 paires de nucléotides successives, et une région II complémentaire au sein de la structure double brin et formée de deux ARN simple brin séparés, la cohésion obtenue grâce aux paires de nucléotides du brin complémentaire II étant augmentée par une liaison chimique supplémentaire et où la liaison chimique est réalisée à une extrémité de la région complémentaire II.

2. Procédé selon la revendication 1, dans lequel l'ARNdb est incorporé dans des structures micellaires, de préférence dans des liposomes.

3. Procédé selon l'une des revendications précédentes, dans lequel le gène cible est exprimé dans des cellules eucaryotes.

4. Procédé selon l'une des revendications précédentes, dans lequel le gène cible est sélectionné parmi: oncogène, gène de cytokine, gène d'Id-protéine, gène de développement, gène de prion.

5. Procédé selon l'une des revendications précédentes, dans lequel le gène cible est une composante d'un virus ou d'un viroïde.

6. Procédé selon la revendication 5, dans lequel le virus est un virus ou un viroïde pathogène humain.

7. Procédé selon la revendication 5, dans lequel le virus ou le viroïde est un virus ou un viroïde pathogène animal.

8. Procédé selon l'une des revendications précédentes, dans lequel les extrémités de l'ARNdb sont modifiées pour contrer une dégradation dans la cellule ou une dissociation en simples brins.

9. Procédé selon l'une des revendications précédentes, dans lequel la cohésion de la région complémentaire II occasionnée par les paires de nucléotides est renforcée par une autre liaison chimique.

10. Procédé selon l'une des revendications précédentes, dans lequel la liaison chimique est formée par une liaison covalente ou ionique, une liaison par pontage d'hydrogène, des interactions hydrophobes, de préférence des interactions Van der Waals ou d'empilement, ou par une coordination ionique métallique.

11. Procédé selon l'une des revendications précédentes, dans lequel la liaison chimique est formée au moyen d'un ou de plusieurs groupes de liaison, les groupes de liaison étant de préférence des chaînes poly(oxyphosphinico-oxy-1,3-propanediol) et/ou polyéthylèneglycol.

12. Procédé selon l'une des revendications précédentes, dans lequel la liaison chimique est formée par des analogues de purine utilisés à la place de la purine dans la région complémentaire II.

13. Procédé selon l'une des revendications précédentes, dans lequel la liaison chimique est formée par des motifs azabenzène introduits dans la région complémentaire II.

14. Procédé selon l'une des revendications précédentes, dans lequel la liaison chimique est formée par des analogues nucléotidiques ramifiés utilisés à la place de nucléotides dans la région complémentaire II.

15. Procédé selon l'une des revendications précédentes, dans lequel on utilise au moins un des groupes suivants pour former la liaison chimique: bleu de méthylène; groupes bifonctionnels, de préférence une bis-(2-chloroéthyl)aminé ; N-acétyl-N'-(p-glyoxyl-benzoyl)-cystamine; 4-thiouracile; psoralène.

16. Procédé selon l'une des revendications précédentes, dans lequel la liaison chimique est formée par des groupes thiophosphoryle appliqués au niveau des extrémités de la région double brin.

17. Procédé selon l'une des revendications précédentes, dans lequel au moins un groupe 2'-hydroxyle des nucléotides de l'ARNdb est remplacé dans la région complémentaire II par un groupe chimique, de préférence un groupe 2'-amino ou 2'-méthyle.

18. Procédé selon l'une des revendications précédentes, dans lequel au moins un nucléotide d'au moins un brin de la région complémentaire II est un "nucléotide fermé" présentant un cycle sucre modifié chimiquement, de préférence par un pont 2'-O, 4'-C-méthylène.

19. Procédé selon l'une des revendications précédentes, dans lequel l'ARNdb est lié à, associé à ou entouré par au moins une protéine enveloppe virale provenant d'un virus, dérivée de celui-ci ou fabriquée par voie synthétique.

20. Procédé selon l'une des revendications précédentes, dans lequel l'ARNdb est complémentaire du transcript d'ARN primaire au traité du gène cible.

21. Procédé selon l'une des revendications précédentes, dans lequel la cellule est une cellule humaine.

22. Procédé selon l'une des revendications précédentes, dans lequel au moins deux ARNdb différents l'un de l'autre sont introduits dans la cellule, un brin de chaque ARNdb étant complémentaire au moins sur certaines portions ou segments de respectivement un d'au moins deux gènes cibles différents.

23. Procédé selon l'une des revendications précédentes, dans lequel un des gènes cible est le gène PKR.

24. Médicament comportant au moins un oligoribonucléotide d'une structure double brin (ARNdb) présentant 15 à 49 paires de base, destiné à inhiber l'expression d'un gène cible donné dans des cellules de mammifères, un brin de l'ARNdb présentant une région I complémentaire au moins sur certains segments du gène cible et qui présente au plus 49 paires de nucléotides successives, et une région II complémentaire au sein de la structure double brin et formée de deux ARN simple brin séparés, la cohésion obtenue grâce aux paires de nucléotides du brin complémentaire II étant augmentée par une liaison chimique supplémentaire et où la liaison chimique est réalisée à une extrémité de la région complémentaire II.

25. Médicament selon la revendication 24, dans lequel l'ARNdb est empaqueté dans des structures micellaires, de préférence des liposomes.

26. Médicament selon l'une des revendications 24 ou 25, dans lequel le gène cible peut être exprimé dans des cellules eucaryotes.

27. Médicament selon l'une des revendications 24 à 26, dans lequel le gène cible est sélectionné parmi: oncogène, gène de cytokine, gène d'Id-protéine, gène de développement, gène de prion.

28. Médicament selon l'une des revendications 24 à 27, dans lequel le gène cible peut être exprimé dans des organismes pathogènes, de préférence dans des plasmodies.

29. Médicament selon l'une des revendications 24 à 28, dans lequel le gène cible est une composante d'un virus ou d'un viroïde.

30. Médicament selon la revendication 29, dans lequel le virus est un virus ou un viroïde pathogène humain.

31. Médicament selon la revendication 29, dans lequel le virus ou le viroïde est un virus ou un viroïde pathogène animal.

32. Médicament selon l'une des revendications 24 à 31, dans lequel les extrémités de l'ARNdb sont modifiées pour contrer une dégradation dans la cellule ou une dissociation en simples brins.

33. Médicament selon l'une des revendications 24 à 32, dans lequel la cohésion de la région complémentaire II occasionnée par les paires de nucléotides est renforcée par une autre liaison chimique.

34. Médicament selon l'une des revendications 24 à 33, dans lequel la liaison chimique est formée par une liaison covalente ou ionique, une liaison par pontage d'hydrogène, des interactions hydrophobes, de préférence des interactions Van der Waals ou d'empilement, ou par une coordination ionique métallique.

35. Médicament selon l'une des revendications 24 à 34, dans lequel la liaison chimique est formée au moyen d'un ou de plusieurs groupes de liaison, les groupes de liaison étant de préférence des chaînes poly-(oxyphosphinico-oxy-1,3-propanediol) et/ou polyéthylèneglycol.

36. Médicament selon l'une des revendications 24 à 35, dans lequel la liaison chimique est formée par des analogues de purine utilisés à la place de la purine dans la région complémentaire II.

37. Médicament selon l'une des revendications 24 à 36, dans lequel la liaison chimique est formée par des motifs azabenzène introduits dans la région complémentaire II.

38. Médicament selon l'une des revendications 24 à 37, dans lequel la liaison chimique est formée par des analogues nucléotidiques ramifiés utilisés à la place de nucléotides dans la région complémentaire II.

39. Médicament selon l'une des revendications 24 à 38, dans lequel on utilise au moins un des groupes suivants pour former la liaison chimique: bleu de méthylène; groupes bifonctionnels, de préférence une bis-(2-chloroéthyl)aminé; N-acétyl-N'-(p-glyoxyl-benzoyl)-cystamine; 4-thiouracile; psoralène.

40. Médicament selon l'une des revendications 24 à 39, dans lequel la liaison chimique est formée par des groupes thiophosphoryle prévus au niveau des extrémités de la région double brin.

41. Médicament selon l'une des revendications 24 à 40, dans lequel au moins un groupe 2'-hydroxyle des nucléotides de l'ARNdb est remplacé dans la région complémentaire II par un groupe chimique, de préférence un groupe 2'-amino ou 2'-méthyle.

42. Médicament selon l'une des revendications 24 à 41, dans lequel au moins un nucléotide d'au moins un brin de la région complémentaire II est un "nucléotide fermé" présentant un cycle sucre modifié chimiquement, de préférence par un pont 2'-O, 4'-C-méthylène.

43. Médicament selon l'une des revendications 24 à 42, dans lequel l'ARNdb est lié à, associé à ou entouré par au moins une protéine enveloppe virale provenant d'un virus, dérivée de celui-ci ou fabriquée par voie synthétique.

44. Médicament selon l'une des revendications 24 à 43, dans lequel l'ARNdb est complémentaire du transcript d'ARN primaire ou traité du gène cible.

45. Médicament selon l'une des revendications 24 à 44, dans lequel la cellule est une cellule humaine.

46. Médicament selon l'une des revendications 24 à 45, dans lequel sont contenus au moins deux ARNdb différents l'un de l'autre, un brin de chaque ARNdb étant complémentaire au moins sur certaines portions ou segments de respectivement un d'au moins deux gènes cibles différents.

47. Médicament selon la revendication 46, dans lequel un des gènes cible est le gène PKR.

48. Utilisation d'un oligoribonucléotide d'une structure double brin (ARNdb) présentant 15 à 49 paires de base pour préparer un médicament, un brin de l'ARNdb présentant une région I complémentaire au moins sur certains segments d'un gène cible prédéfini dans des cellules de mammifères et qui présente au plus 49 paires de nucléotides successives, et une région II complémentaire au sein de la structure double brin et formée de deux ARN simple brin séparés, la cohésion obtenue grâce aux paires de nucléotides du brin complémentaire II étant augmentée par une liaison chimique supplémentaire et où la liaison chimique est réalisée à une extrémité de la région complémentaire II.

49. Utilisation selon la revendication 48, dans laquelle l'ARNdb est empaqueté dans des structures micellaires, de préférence des liposomes.

50. Utilisation selon l'une des revendications 48 ou 49, dans laquelle le gène cible peut être exprimé dans des cellules eucaryotes.

51. Utilisation selon l'une des revendications 48 à 50, dans laquelle le gène cible est sélectionné parmi: oncogène, gène de cytokine, gène d'Id-protéine, gène de développement, gène de prion.

52. Utilisation selon l'une des revendications 48 à 51, dans laquelle le gène cible peut être exprimé dans des organismes pathogènes, de préférence dans des plasmodies.

53. Utilisation selon l'une des revendications 48 à 52, dans laquelle le gène cible est une composante d'un virus ou d'un viroïde.

54. Utilisation selon la revendication 53, dans laquelle le virus est un virus ou un viroïde pathogène humain.

55. Utilisation selon la revendication 53, dans laquelle le virus ou le viroïde est un virus ou un viroïde pathogène animal.

56. Utilisation selon l'une des revendications 48 à 55, dans laquelle les extrémités de l'ARNdb sont modifiées pour contrer une dégradation dans la cellule ou une dissociation en simples brins.

57. Utilisation selon l'une des revendications 48 à 56, dans laquelle la cohésion de la région complémentaire II occasionnée par les paires de nucléotides est augmentée par une autre liaison chimique.

58. Utilisation selon l'une des revendications 48 à 57, dans laquelle la liaison chimique est formée par une liaison covalente ou ionique, une liaison par pontage d'hydrogène, des interactions hydrophobes, de préférence des interactions Van der Waals ou d'empilement, ou par une coordination ionique métallique.

59. Utilisation selon l'une des revendications 48 à 58, dans laquelle la liaison chimique est formée au moyen d'un ou de plusieurs groupes de liaison, les groupes de liaison étant de préférence des chaînes poly-(oxyphosphinico-oxy-1,3-propanediol) et/ou polyéthylèneglycol.

60. Utilisation selon l'une des revendications 48 à 59, dans laquelle la liaison chimique est formée par des analogues de purine utilisés à la place de la purine dans la région complémentaire II.

61. Utilisation selon l'une des revendications 48 à 60, dans laquelle la liaison chimique est formée par des motifs azabenzène introduits dans la région complémentaire II.

62. Utilisation selon l'une des revendications 48 à 61, dans laquelle la liaison chimique est formée par des analogues nucléotidiques ramifiés utilisés à la place de nucléotides dans la région complémentaire II.

63. Utilisation selon l'une des revendications 48 à 62, dans laquelle on utilise au moins un des groupes suivants pour former la liaison chimique: bleu de méthylène; groupes bifonctionnels, de préférence une bis-(2-chloroéthyl)aminé ; N-acétyl-N'-(p-glyoxyl-benzoyl)-cystamine; 4-thiouracile; psoralène.

64. Utilisation selon l'une des revendications 48 à 63, dans laquelle la liaison chimique est formée par des groupes thiophosphoryle appliqués au niveau des extrémités de la région double brin.

65. Utilisation selon l'une des revendications 48 à 64, dans laquelle au moins un groupe 2'-hydroxyle des nucléotides de l'ARNdb est remplacé dans la région complémentaire II par un groupe chimique, de préférence un groupe 2'-amino- ou 2'-méthyle.

66. Utilisation selon l'une des revendications 48 à 65, dans laquelle au moins un nucléotide d'au moins un brin de la région complémentaire II est un "nucléotide fermé" présentant un cycle sucre modifié chimiquement, de préférence par un pont 2'-O, 4'-C-méthylène.

67. Utilisation selon l'une des revendications 48 à 66, dans laquelle l'ARNdb est lié à, associé à ou entouré par au moins une protéine enveloppe virale provenant d'un virus, dérivée de celui-ci ou fabriquée par voie synthétique.

68. Utilisation selon l'une des revendications 48 à 67, dans laquelle l'ARNdb est complémentaire du transcript d'ARN primaire ou traité du gène cible.

69. Utilisation selon l'une des revendications 48 à 68, dans laquelle la cellule est une cellule humaine.

70. Utilisation selon l'une des revendications 48 à 69, dans laquelle au moins deux ARNdb différents l'un de l'autre sont utilisés, un brin de chaque ARNdb étant complémentaire au moins sur certaines portions ou segments de respectivement un d'au moins deux gènes cibles différents.

71. Utilisation selon la revendication 70, dans laquelle un des gènes cible est le gène PKR.

72. Utilisation selon l'une des revendications 48 à 71, dans laquelle le médicament peut être injecté dans le flux sanguin ou dans l'interstitium de l'organisme sur lequel est réalisée la thérapie.


### SEQUENZPROTOKOLL

<110> Kreutzer Dr., Roland
   Limmer Dr., Stephan
<120> Verfahren und Medikament zur Hemmung der Expression eines vorgegebenen Gens
<130> 400968
<140>
   <141>
<150> 199 03 713.2
   <151> 1999-01-30
<150> 199 56 568.6
   <151> 1999-11-24
<160> 8
<170> Patentin Ver. 2.1
<210> 1
   <211> 45
   <212> DNA
   <213> Künstliche Sequenz
<220>
   <223> Beschreibung der künstlichen Sequenz:
   EcoRI-Schnittstelle, T7-RNA-polymerasepromotor
<400> 1
   **ggaattctaa tacgactcac tatagggcga tcagatctct agaag 45**
<210> 2
   <211> 50
   <212> DNA
   <213> Künstliche Sequenz
<220>
   <223> Beschreibung der künstlichen Sequenz:
   BamHI-Schnittstelle, SP6-RNA-Polymerasepromotor
<400> 2
   **gggatccatt taggtgacac tatagaatac ccatgatcgc gtagtcgata 50**
<210> 3
   <211> 340
   <212> RNA
   <213> Künstliche Sequenz
<220>
   <223> Beschreibung der künstlichen Sequenz: RNA, die einer Sequenz aus der "positive control DNA" des HeLaScribe Nuclear Extract in vitro Transkriptionskits der Firma Promega entspricht
<400> 3
   ucagaucucu agaagcuuua augcgguagu uuaucacagu uaaauugcua acgcagucag 60
   gcaccgugua ugaaaucuaa caaugcgcuc aucgucaucc ucggcaccgu cacccuggau 120
   gcuguaggca uaggcuuggu uaugccggua cugccgggcc ucuugcggga uaucguccau 180
   uccgacagca ucgccaguca cuauggcgug cugcuagcgc uauaugcguu gaugcaauuu 240
   cuaugcgcac ccguucucgg agcacugucc gaccgcuuug gccgccgccc aguccugcuc 300
   gcuucgcuac uuggagccac uaucgacuac gcgaucaugg 340
<210> 4
   <211> 363
   <212> DNA
   <213> Künstliche Sequenz
<220>
   <223> Beschreibung der künstlichen Sequenz: DNA, die einer Sequenz aus der "positive control DNA" des HeLaScribe Nuclear Extract in vitro Transkriptionskits der Firma Promega entspricht
<400> 4
   tcagatctct agaagcttta atgcggtagt ttatcacagt taaattgcta acgcagtcag 60
   gcaccgtgta tgaaatctaa caatgcgctc atcgtcatcc tcggcaccgt caccctggat 120
   gctgtaggca taggcttggt tatgccggta ctgccgggcc tcttgcggga tatcgtccat 180
   tccgacagca tcgccagtca ctatggcgtg ctgctagcgc tatatgcgtt gatgcaattt 240
   ctatgcgcac ccgttctcgg agcactgtcc gaccgctttg gccgccgccc agtcctgctc 300
   gcttcgctac ttggagccac tatcgactac gcgatcatgg cgaccacacc cgtcctgtgg 360
   atc 363
<210> 5
   <211> 315
   <212> RNA
   <213> Künstliche Sequenz
<220>
   <223> Beschreibung der künstlichen Sequenz: Sequenz aus dem YFP-Gen
<400> 5
   auggugagca agggcgagga gcuguucacc gggguggugc ccauccuggu cgagcuggac 60
   ggcgacguaa acggccacaa guucagcgug uccggcgagg gcgagggcga ugccaccuac 120
   ggcaagcuga cccugaaguu caucugcacc accggcaagc ugcccgugcc cuggcccacc 180
   cucgugacca cccugaccua cggcgugcag ugcuucagcc gcuaccccga ccacaugaag 240
   cagcacgacu ucuucaaguc cgccaugccc gaaggcuacg uccaggagcg caccaucuuc 300
   uucaaggacg acggc 315
<210> 6
   <211> 52
   <212> DNA
   <213> Künstliche Sequenz
<220>
   <223> Beschreibung der künstlichen Sequenz: EcoRI-Schnittstelle, T7-RNA-Polymerasepromotor, komplementärer Bereich zum YFP-Gen
<400> 6
   **ggaattctaa tacgactcac tatagggcga atggtgagca agggcgagga gc 52**
<210> 7
   <211> 53
   <212> DNA
   <213> Künstliche Sequenz
<220>
   <223> Beschreibung der künstlichen Sequenz: BamHI-Schnittstelle, SP6-RNA-Polymerasepromotor, komplementärer Bereich zum YFP-Gen
<400> 7
   **gggatccatt taggtgtgacac tatagaatac gccgtcgtcc ttgaagaaga tgg 53**
<210> 8
   <211> 21
   <212> RNA
   <213> Künstliche Sequenz
<220>
   <223> Beschreibung der künstlichen Sequenz: RNA, die einer Sequenz aus dem YFP-Gen entspricht
<400> 8
   **ucgagcugga cggcgacgua a 21**